# EUROPEAN PATENT SPECIFICATION

(11) **EP 0 300 657 B1**
(45) Date of publication and mention of the grant of the patent: **31.03.1993**
(21) Application number: 88306291.1
(22) Date of filing: 08.07.1988
(51) Int. Cl.: C07D 205/08, C07D 405/06, C07F 7/18, C07F 7/08, C07F 3/02

(54) **4-substituted 3-oxobutanoate ester derivatives**
4-Substituierte 3-Oxobutanoat-Esterderivate
Dérivés d'ester 3-oxobutanoique 4-substitué

(30) Priority: 17.07.1987 US 75009
(43) Date of publication of application: 25.01.1989
(73) Proprietor: Merck & Co., Inc., Rahway New Jersey 07065-0900 (US)
(72) Inventor: Fuentes, Lelia M., Westfield, N.J. 07090 (US); Hazen, George G., Perth Amboy, N.J. 08861 (US); Tschaen, David M., Aberdeen, N.J. 07747 (US); Weinstock, Leonard M., Belle Mead, N.J. 08502 (US)
(74) Representative: Cole, William Gwyn

(56) References cited:
- EP-A- 0 026 817
- EP-A- 0 037 080
- US-A- 4 262 010
- US-A- 4 282 148
- US-A- 4 312 871
- ANGEWANDTE CHEMIE, International Edition in English, vol. 18, no. 1, 1979, pages 72-74; D.W. BROOKS et al.: "C-acylation under virtually neutral conditions"
- JOURNAL OF ORGANIC CHEMISTRY, vol. 50, no. 15, 26th July 1985, pages 2622-2624, American Chemical Society; M.W. RATHKE et al.: "Procedures for the acylation of diethyl malonate and ethyl acetoacetate with acid chlorides using tertiary amine bases and magnesium chloride"

## Description

### BACKGROUND OF THE INVENTION

### (a) Field of the Invention

This invention relates to a process for preparing 4-substituted 3-oxobutanoate ester derivatives by reaction of essentially anhydrous magnesium malonate monoester salts with activated acyl compounds. In particular, this invention relates to a process for preparing essentially anhydrous magnesium malonate monoester salts and for using said salts to prepare certain compounds of Formula I (see below) having utility as intermediates in the preparation of carbapenem antibiotics.

### (b) Prior Art

Numerous methods known in the art are available for C-acylation at the methylene carbon of malonic acid derivatives. For example, Brooks, Lu, and Masamune have reported a neutral C-acylation method that involves reaction of acyl imidazolides with magnesium malonate monoester salts, followed by a decarboxylation to form β-ketoesters. Angew. Chem. Int. Ed. Engl., 18, 72-74 (1979). Formation of the magnesium salts by reaction of magnesium ethoxide with malonate monoesters necessarily releases water as a byproduct. Drying the magnesium salt for the C-acylation procedure typically involves azeotropic drying and can be complicated by possible ester interchange and decarboxylation. In contrast to the method of Brooks et al., the process of the present invention generates essentially anhydrous magnesium malonate monoester salts that can be used in situ without additional drying.

Rathke and Corvan have reported a C-acylation method that involves reaction of acyl halides with malonate diesters in the presence of magnesium chloride and trialkylamines but does not include a decarboxylation step. J. Org. Chem., 50, 2622-2624 (1985). The Rathke and Corvan report does not suggest the preparation of β-ketoesters from malonate monoesters.

Certain β,4-dioxo-2-azetidinebutanoic acid derivatives that fall within the general scope of compounds of Formula I have been prepared by C-acylation of magnesium monoester salts using certain activated 4-oxo-2-azetidineacetyl derivatives. See U.S. Patent Nos. 4,414,155, 4,360,684, 4,350,631, 4,312,871, 4,282,148, 4,273,709, and 4,262,010; and Shih et al., Heterocycles, 21, 29-40 (1984). (For further disclosures, see also U.S. Patent Nos. 4,467,107, 4,454,332, 4,499,278, 4,349,687, 4,344,885, 4,287,123, and 4,269,772.) The cited references, however, do not disclose nor suggest the present novel process for in situ generation and use of anhydrous magnesium malonate monoester salts.

### SUMMARY OF THE INVENTION

Applicants have discovered an advantageous process for preparing 4-substituted 3-oxobutanoate ester derivatives of Formula I 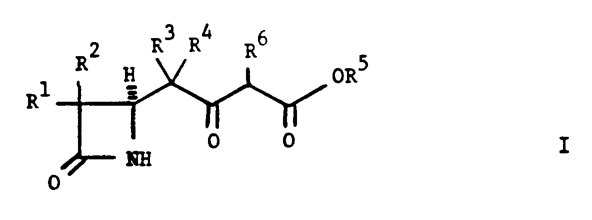
wherein R¹ and R² are independently:
a) hydrogen;
b) C₁-C₄ alkyl;
c) C₁-C₄ alkyl substituted at the 1-position with a substituent selected from the group consisting of:
   i) hydroxy;
   ii) tris (C₁-C₄ alkyl)silyloxy; or
   iii) allyloxycarbonyloxy; or
d) fluorinated C₁-C₄ alkyl; with the proviso that at least one of R₁ and R₂ is hydrogen;
R³ and R⁴ are independently;
a) hydrogen; or
b) C₁-C₄ alkyl;
R⁵ is:
a) benzyl or benzyl substituted in the benzene ring with one to three substituents selected from the group consisting of:
   i) NO₂;
   ii) halogen; or
   iii) C₁-C₄ alkyl;
b) C₁-C₄ alkyl or C₁-C₄ alkyl
   substituted with one or more substituents selected from the group consisting of:
   i) halogen; or
   ii) tris(C₁-C₄ alkyl)silyl; or
c) C₃-C₆ alkenyl or C₃-C₆ alkenyl substituted with a substituent selected from the group consisting of:
   i) phenyl;
   ii) naphthyl; or
   iii) biphenylyl; and
R⁶ is:
a) hydrogen; or
b) COOR⁷ such that R⁵ and R⁷ taken together are -C(CH₃)₂-.

In particular, applicants have discovered a novel process in which a substantially anhydrous magnesium malonate monoester salt is generated and used in situ to prepare compounds of Formula I. More specifically, applicants have discovered a process for preparing compounds of Formula I comprising:
a) reacting at least about one part to about three parts by moles of a malonate derivative of the formula 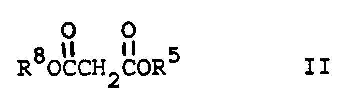 wherein R⁵ is defined above and R⁸ is hydrogen or wherein R⁵ and R⁸ taken together are -C(CH₃)₂-,
   with about one part by moles of a magnesium dihalide and about two parts by moles of an amine base in an organic solvent; and
b) adding about one part to about two parts by moles of an activated acyl compound of the formula

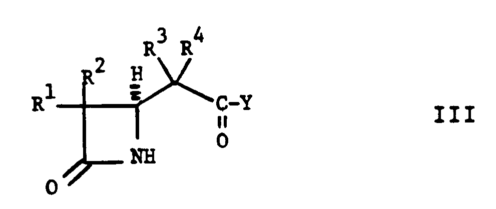

wherein R¹, R², R³, and R⁴ are defined as above and Y is halogen, 1-imidazolyl, or C₁-C₅ acyloxy.

Although the structure shown for Formula I indicates one tautomeric form, it is understood that this representation is for convenience only and that the scope of this invention includes as equivalents all tautomeric forms of the compounds prepared by the process of this invention.

The term "C₁-C₄ alkyl" refers to straight or branched chain aliphatic hydrocarbon groups having from 1 to 4 carbon atoms, also referred to as lower alkyl. Examples of C₁-C₄ alkyl are methyl, ethyl, propyl, butyl, and the isomeric forms thereof.

Substitution at the 1-position of C₁-C₄ alkyl refers to C₁-C₄ alkyl bearing a substituent on the carbon atom most proximately attached to the β-lactam function. For example, 1-hydroxy substituted C₁-C₄ alkyl includes hydroxymethyl, 1-hydroxyethyl, 1-hydroxypropyl, 1-hydroxybutyl, 1-hydroxy-2-methylpropyl, and their optical isomer forms. Similar substitution is possible for the tris(C₁-C₄ alkyl)silyloxy group. For example, 1-tris(C₁-C₄ alkyl)silyloxy-substituted C₁-C₄ alkyl includes (trimethylsilyloxy)methyl, 1-(trimethylsilyloxy)ethyl, (t-butyldimethylsilyloxy)methyl, 1-(t-butyldimethylsilyloxy)ethyl, and the like.

The term "C₁-C₄ fluorinated alkyl" refers to C₁-C₄ alkyl in which one or more hydrogen atoms are replaced with fluorine atoms. Examples of C₁-C₄ fluorinated alkyl are fluoromethyl, difluoromethyl, trifluoromethyl, 1- or 2-fluoroethyl, 1,1-difluoroethyl, 2,2,2-trifluoroethyl, perfluoroethyl; other similarly monofluorinated, polyfluorinated, and perfluorinated ethyl, propyl, and butyl groups; and the isomeric forms thereof.

The term "C₃-C₆ alkenyl" refers to straight or branched chain hydrocarbon groups having from 3 to 6 carbon atoms and possessing one carbon-carbon double bond. As used herein, C₃-C₆ alkenyl preferably does not include such groups in which a double-bond containing carbon is attached directly to the carboxy oxygen of compounds of Formula I. Examples of C₃-C₆ alkenyl are allyl; 2- or 3-butenyl; 2-, 3-, or 4-pentenyl; 2-, 3-, 4-, or 5-hexenyl; and isomeric forms thereof.

The term "C₁-C₅ acyloxy" as used herein refers to formyloxy, straight or branched chain C₂-C₅ alkanoyloxy, or straight or branched chain C₂-C₅ alkoxycarbonyloxy. Examples of C₂-C₅ alkanoyloxy include acetoxy, propanoyloxy, butanoyloxy, pentanoyloxy, and isomeric forms thereof. Examples of C₂-C₅ alkoxycarbonyloxy include methoxycarbonyloxy, ethoxycarbonyloxy, propoxycarbonyloxy, butoxycarbonyloxy, and the isomeric forms thereof.

Examples of halogen are fluorine, chlorine, bromine, and iodine.

### DESCRIPTION OF THE INVENTION

The process of this invention may be effectuated by the general procedures illustrated in the following Schemes. Unless otherwise specified, the various substituents are defined as for Formula I, above. Scheme A illustrates a general method for effectuating the process of this invention for compounds of Formula I in which R⁶ is hydrogen. 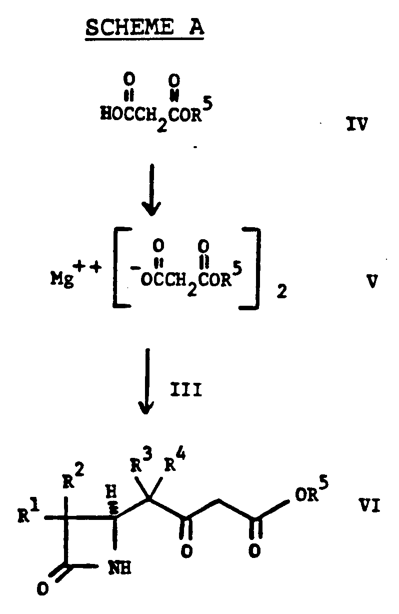
Malonate monoesters of Formula IV (that is, Formula II in which R⁸ is hydrogen) are converted to substantially anhydrous preparations of magnesium malonate monoester salts of Formula V by reaction with a magnesium dihalide and a suitable amine base in a substantially anhydrous suitable organic solvent. The molar quantity of malonate esters IV must at least equal those of the magnesium halide and may range up to large excesses. However, the normal range is from about one-fold up to about three-fold, preferably about two-fold. A preferred magnesium dihalide is magnesium chloride. Suitable amine bases are organic amines and other nitrogen-containing organic bases that facilitate the formation of the magnesium malonate monoester salts and that are sufficiently basic to prevent the reaction medium from becoming acidic but which do not themselves form significant quantities of byproducts by chemical reaction with other reagents. Suitable amine bases include tertiary amines, hindered secondary amines, and nitrogen-containing heteroaromatic compounds. Suitable tertiary amines include trialkylamines, such as triethylamine and tributylamine; N-substituted saturated heterocyclic compounds, such as N-methylmorpholine, N-methylpiperidine, and N,N-dimethylpiperazine; polybasic tertiary amines, such as N,N,N,N-tetramethylethylenediamine and N,N,N,N-tetramethylpropylenediamine; and other tertiary amines known in the art. Suitable hindered secondary amines include 2,2,6,6-tetramethylpiperidine and other hindered secondary amines known in the art. Suitable nitrogen-containing heteroaromatic compounds include pyrrole, 1-methylpyrrole, pyridine, pyrazine, pyrimidine, pyridazine, quinoline, isoquinoline, imidazole, 1-methylimidazole, pyrazole, 1-methylpyrazole, 4-(dimethylamino)pyridine, and other such nitrogen-containing heteroaromatic compounds known in the art. Preferred amine bases include trialkylamines, preferably triethylamine, and imidazole. Suitable organic solvents are non-protic organic liquids in which reactants may be dissolved or suspended but which are otherwise essentially chemically inert. Examples of suitable organic solvents include alkanes and cycloalkanes; ethers and cyclic ethers, such as diethyl ether, tetrahydrofuran, tetrahydropyran; aromatic hydrocarbons; halocarbons, such as chloroform, dichloromethane, ethylene dichloride; N,N-disubstituted amides, such as dimethylformamide, dimethylacetamide; N-substituted lactams, such as N-methylpyrrolidinone, N-methylpiperidinone; cyanoalkanes, such as acetonitrile, propionitrile; and other organic solvents known in the art. Preferred organic solvents include tetrahydrofuran, dimethylformamide, and acetonitrile. The solvents are used in substantially anhydrous form (after drying by methods well known in the art), and the salts of Formula V are prepared in apparatus that reasonably excludes external moisture.

Compounds of Formula VI (that is, Formula I in which R⁶ is hydrogen) are made by allowing a suitably activated acyl compound of Formula III to react with a magnesium malonate monoester salt of Formula IV prepared by the general method described above, preferably in situ in the same solution used to generate the salt of Formula IV. Compare Angew. Chem. Int. Ed. Engl., 18, 72-74 (1979). Since the acyl compounds III are typically more difficult to prepare than are the magnesium salts, a less than equimolar amount of compounds III relative to malonate compounds IV is normally used. A preferred quantity is one molar part of compound III to two molar parts of malonate compound IV. Suitably activated acyl compounds of Formula III are those in which the group Y is a leaving group of a type known in the art for acylation reactions. For example, Y may be a halogen, preferably chlorine; a nitrogen-containing heteroaromatic group, preferably derived from an N-hydrogen substituted heteroaromatic compound such as imidazole; an acyloxy group capable of forming a mixed anhydride, such as formyloxy; and other such acyl-activating groups known in the art. A preferred group Y is 1-imidazolyl. Compounds of Formula III wherein Y is imidazolyl may be prepared by methods known in the art, for example by using carbonyldiimidazole or a reaction of a mixed anhydride intermediate with imidazole. See, for example, Shih et al., Heterocycles, 21, 29-40 (1984) and U.S. Patent Nos. 4,499,278, 4,360,684, 4,350,631, 4,312,871, 4,282,148, 4,273,709, and 4,262,010. During the course of the reaction, the substituted malonate intermediate formed by acylation (that is, Formula I in which R⁶ is -COOH or a magnesium salt thereof) decarboxylates to yield the desired product of Formula VI. The acylation and decarboxylation reaction may be performed over a broad temperature range but preferably at from about 0°C to about 50°C, most preferably at about room temperature.

Scheme B illustrates a preferred method for effectuating the process of this invention for compounds of Formula I in which R⁶ and R⁵ together form a cyclic structure as in Formula VIII. 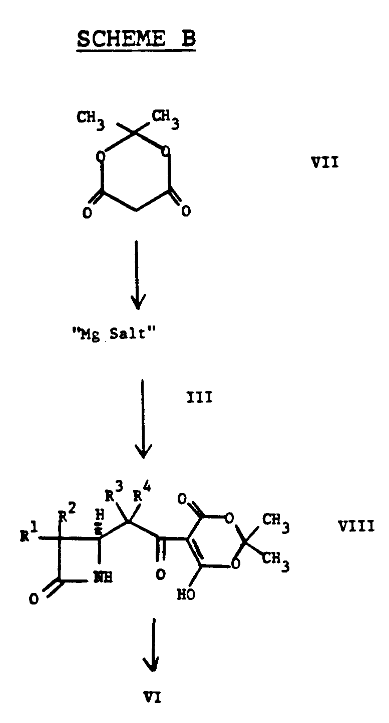
The cyclic malonate ester known as Meldrum's acid, and having the Formula VII (that is, Formula II in which R⁵ and R⁸ taken together are -C(CH₃)₂-), can be converted to a substantially anhydrous magnesium salt using essentially the same methods described above for Scheme A. Normally equimolar quantities of Meldrum's acid and the magnesium halide are used. The nature of the magnesium salt may be dependent somewhat on the stoichiometry used but presumably has the formula 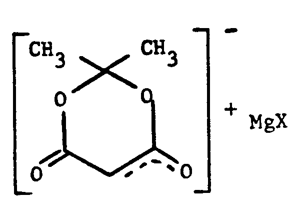
wherein X is halogen and the dotted line indicates delocalized bonding. For the purposes of the process of this invention the precise structure of the magnesium salt is not critical to the extent that the relative amounts of Meldrum's acid and the activated acyl compound of Formula III are known. As described in Scheme A, the magnesium salt is allowed to react with an activated acyl compound of Formula III to form compounds of this invention of Formula VIII.

Compounds of Formula VIII provide an alternative means for preparing corresponding open-chain compounds of Formula VI (that is, Formula I in which R⁶ is hydrogen; see Scheme A). Reaction of a compound of Formula VIII with a nucleophilic alcohol of the formula R⁵OH in a suitable solvent, which may include the alcohol R⁵OH itself, at temperatures ranging from about 40°C to about 100°C yields the corresponding compound of Formula VI. During the course of the reaction, the substituted malonate intermediate formed during ring opening (that is, Formula I in which R⁶ is -COOH) decarboxylates to form the desired product of Formula VI. Where a solvent other than R⁵OH is used, suitable solvents include organic liquids in which reactants may be dissolved or suspended and which, if necessary, can be heated sufficiently to induce decarboxylation but which are otherwise chemically inert. Examples of suitable solvents include alkanes and cycloalkanes; ethers and cyclic ethers; aromatic hydrocarbons; halocarbons; N,N-disubstituted amides, such as dimethylformamide; N-substituted lactams, such as N-methylpyrrolidinone; and other solvents known in the art. Preferred solvents include alcohols of the formula R⁵OH.

The preferred embodiments of this invention include a process for preparing compounds of the following general structure: 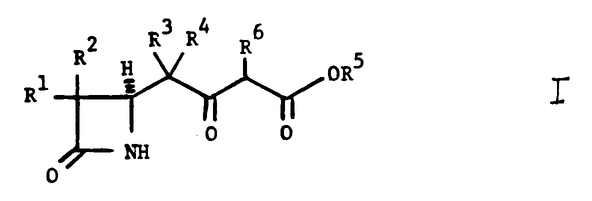
wherein one of R¹ or R² is hydrogen and the other of R¹ or R² is 1-hydroxyethyl or 1-(t-butyldimethylsilyloxy)ethyl;
one of R³ or R⁴ is hydrogen and the other of R³ or R⁴ is hydrogen or C₁-C₄ alkyl;
R⁵ is:
a) benzyl or benzyl substituted in the benzene ring with one to three substituents selected from the group consisting of:
   i) NO₂;
   ii) halogen; or
   iii) C₁-C₄ alkyl; or
b) C₁-C₄ alkyl or C₁-C₄ alkyl
   substituted with one or more substituents selected from the group consisting of:
   i) halogen; or
   ii) tris(C₁-C₄ alkyl)silyl; or
c) C₃-C₆ alkenyl or C₃-C₆ alkenyl substituted with phenyl; and
R⁶ is:
a) hydrogen; or
b) COOR⁷ such that R⁵ and R⁷ taken together are -C(CH₃)₂-;
comprising:
a) reacting at least about one part to about three parts by moles of a malonate derivative of the formula 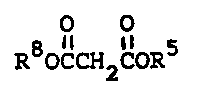 wherein R⁵ is defined as above and R⁸ is hydrogen or wherein R⁵ and R⁸ taken together are -C(CH₃)₂-,
   with about one part by moles of magnesium chloride and about two parts by moles of a trialkylamine or imidazole in an organic solvent selected from the group consisting of tetrahydrofuran, acetonitrile, and dimethylformamide; and
b) adding about one part by moles of an activated acyl compound of the formula 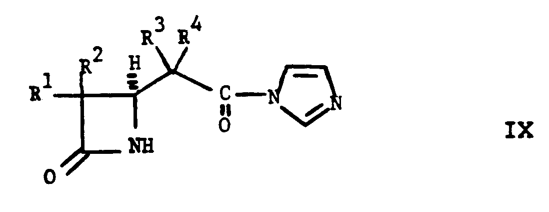 wherein R¹, R², R³, and R⁴ are defined as above for Formula I.

A more preferred embodiment of this invention includes a process for preparing compounds of the following general structure: 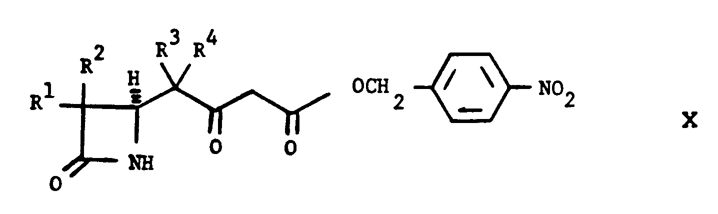
wherein one of R¹ or R² is hydrogen and the other of R¹ or R² is 1-hydroxyethyl or 1-(t-butyldimethylsilyloxy)ethyl; and one of R³ or R⁴ is hydrogen and the other of R³ or R⁴ is hydrogen or C₁-C₄ alkyl;
comprising:
a) reacting about two parts by moles of 4-nitrobenzyl malonate of the formula 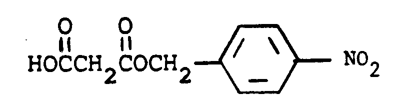 with about one part by moles of magnesium chloride and about two parts by moles of triethylamine in tetrahydrofuran or dimethylformamide; and
b) adding about one part by moles of an activated acyl compound of the formula 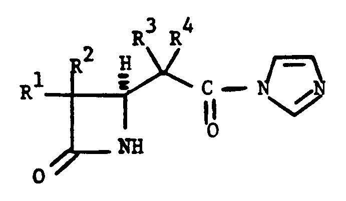 wherein R¹, R², R³, and R⁴ are defined as above for Formula X.

The following examples further illustrate details for the process of this invention. All temperatures are degrees Celsium unless otherwise noted. Except as otherwise indicated, compounds prepared in Examples 5-15 were characterized by comparison with independently prepared authentic samples.

### DESCRIPTION OF THE PREFERRED EMBODIMENTS

### Example 1

### Magnesium 4-nitrobenzyl malonate

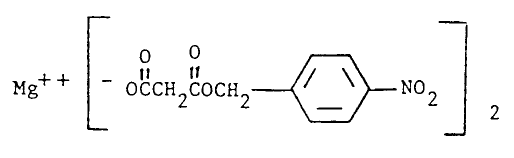

A stirred mixture of 4-nitrobenzyl malonate (4.70 g, 19.68 mmole) and anhydrous magnesium chloride (1.0 g, 10.5 mmole) in sieve-dried dimethylformamide (20 ml) was cooled to ca. -20°. A solution of triethylamine (2.07 g 20.46 mmole) in sieve-dried dimethylformamide (4.0 ml) was added dropwise with stirring while maintaining the temperature below -10°. The mixture was stirred at ca. 0° for an additional 15 minutes. The desired title compound was used without isolation in subsequent reactions. Structure assignment was supported by the Fourier transform infrared spectrum of the reaction mixture (by comparison with an authentic sample).

### EXAMPLE 2

### Magnesium 2-(trimethylsilyl)ethyl malonate

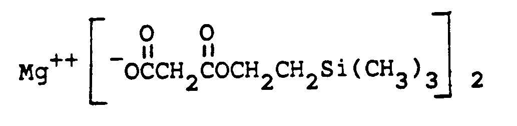

A mixture of Meldrum's acid (1 g) and 2-(trimethylsilyl)ethanol (0.94 ml) in sieve-dried tetrahydrofuran (8 ml) was heated at reflux for 24 hours. Concentration in vacuo yielded 2-(trimethylsilyl)ethyl malonate as a colorless oil. The title compound was then prepared by the method of Example 1 using 2-(trimethylsilyl)ethyl malonate instead of 4-nitrobenzyl malonate and tetrahydrofuran at 0° rather than dimethylformamide at -20°C, with appropriate adjustment of quantities of the reagents. The magnesium salt was used without isolation in subsequent reactions.

### Example 3

### Magnesium 2,2,2-trichloroethyl malonate

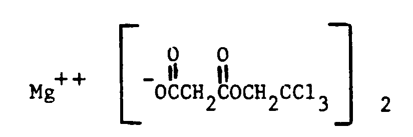

A mixture of Meldrum's acid (2 g) and 2,2,2-trichloroethanol (1.23 ml) in sieve-dried tetrahydrofuran (8 ml) was heated at reflux for 24 hours. Partial concentration in vacuo produced a small amount of a precipitate that was removed by filtration. Concentration of the filtrate yielded 2,2,2-trichloroethyl malonate. The title compound was then prepared by the method of Example 1 using 2,2,2-trichloroethyl malonate instead of 4-nitrobenzyl malonate and tetrahydrofuran at 0°C instead of dimethylformamide at -20°C, with appropriate adjustment of quantities of the reagents. The magnesium salt was used without isolation in subsequent reactions.

### Example 4

### Magnesium cinnamyl malonate

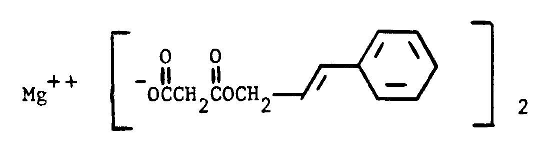

A mixture of Meldrum's acid (3 g) and cinnamyl alcohol (2.79 g) in tetrahydrofuran (15 ml) was heated at reflux for 24 hours. Concentration in vacuo yielded cinnamyl malonate. The title compound was then prepared by the method of Example 1 using cinnamyl malonate instead of 4-nitrobenzyl malonate and tetrahydrofuran at 0° instead of dimethylformamide at -20°, with appropriate adjustment of quantities of the reagents. The magnesium salt was used without isolation in subsequent reactions.

### Example 5

### [2α(S*),3β(R*)]-3-[1-[[1,1-Dimethylethyl)dimethylsilyl]oxy]ethyl]-γ-methyl-β,4-dioxo-2-azetidine butanoic acid (4-nitrophenyl)methyl ester

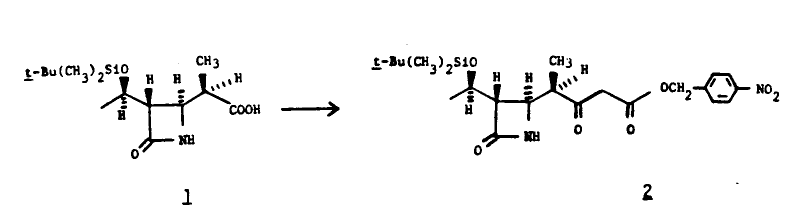

A solution of 0.956 M carbonyldiimidazole in dimethylformamide (1.35 ml, 1.291 mmole) was added at room temperature under nitrogen to the carboxylic acid 1 (0.354 g, 1.174 mmoles; prepared as described by Shih et al., Heterocycles, 21, 29-40 (1984)), with the careful exclusion of moisture. After the mixture was stirred for about 30 minutes, during which time the acyl imidazole derivative formed, magnesium 4-nitrobenzyl malonate (2.35 mmole in dimethylformamide and prepared as described in Example 1) was added dropwise at room temperature. The mixture was then warmed to ca. 50° and stirred for one hour. The crude mixture was purified by flash chromatography on silica gel (using 2:1 ethyl acetate-hexanes as eluent) to yield 533 mg (ca. 94%) of the title compound 2 as a crystalline solid.

### Example 6

### [2α(S*),3β(R*)]-5-[2-[3-[1-[[(1,1-Dimethylethyl)dimethylsilyl]oxy]ethyl]-4-oxo-2-azetidinyl]-1-oxo-propyl]-6-hydroxy-2,2-dimethyl-4H-1,3-dioxin-4-one

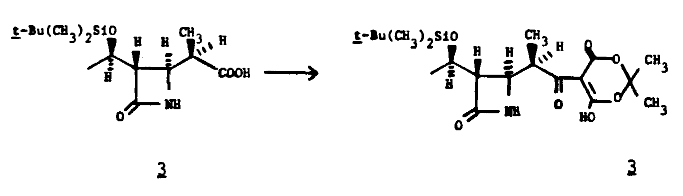

A solution of 0.956 M carbonyldiimidazole in dimethylformamide (11.0 ml, 10.5 mmole) was added at room temperature under nitrogen to the carboxylic acid 1 (2.873 g, 9.52 mmole), and the mixture was stirred for 30 minutes. In a separate flask containing Meldrum's acid (1.716 g, 11.90 mmole) and magnesium chloride 1.127 g, 11.82 mmole) in acetonitrile under nitrogen (25 ml) was added dropwise triethylamine (2.43 g, 24.00 mmole), and the mixture was stirred for about 45 minutes. The solution containing the imidazole activated carboxylic acid was added to the slurry containing the magnesium salt of Meldrum's acid, and the mixture was stirred at room temperature for about six hours. The mixture was cooled to 0° and acidified with 1N hydrochloric acid, and the mixture was then extracted with dichloromethane (2 x 50 ml). The organic layer was washed sequentially with saturated aqueous sodium bicarbonate (50 ml) and water (50 ml), dried over magnesium sulfate, and concentrated in vacuo to yield 3.87 of crude title compound 3.

### Example 7

### [2α,3β(R*)]-3-[1-[[(1,1-Dimethylethyl)dimethylsilyl]oxy]ethyl]-β,4-dioxo-2-azetidinebutanoic acid 2-propenyl ester

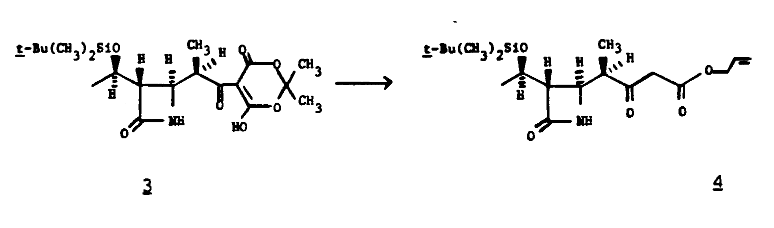

To the crude title product 3 of Example 6 was added allyl alcohol (1.0 ml, ca. 15.00 mmole). The mixture was heated at 70° for one hour, another 1.0 ml of allyl alcohol was added, and the mixture was heated at 70° for another hour. The mixture was cooled to room temperature and concentrated in vacuo. The residue was purified by flash chromatography on silica gel (using 20% by volume of ethyl acetate-hexanes as eluent) to yield 2.60 g (ca. 76%) of the title compound 4. Structure assignment was supported by the nmr and infrared spectra, by chemical ionization mass spectrometry, and by elemental analysis:
- ¹H nmr (CDCl₃; 250 MHz):: δ (ppm) 0.06 (pair s, 6H); 0.86 (s, 9H); 1.20 (d, J=6.25 Hz, 3H); 2.76 (dd, J=2.8, 5.1 Hz, 1H); 2.78 (dd, J=9.9, 18.0 Hz, 1H); 3.03 (dd, J=3.4, 18.0 Hz, 1H); 3.49 (s, 2H); 3.96 (m, 1H); 4.15 (m, 1H); 4.63 (m, 2H); 5.15 (m, 2H); 5.90 (m, 1H); 6.15 (bs, 1H; NH).
- IR (thin film):: 3250, 2925, 1750, 1735, 1715, 1650, 1255, 1140, 1090, 830 cm⁻¹.
- MS (C.I.):: 370 (MH⁺), 354, 312, 269, 170.
- Analysis. Calc'd. for C₁₉H₃₂NO₅Si:: C, 59.64; H, 8.44; N, 3.66. Found: C, 59.79, H, 8.47; N, 3.75.

### Example 8

### [2α,3β(R*)]-3-[1-[[(1,1-Dimethylethyl)dimethylsilyl]oxy]ethyl]-β,4-dioxo-2-azetidinebutanoic acid 2-(trimethylsilyl)ethyl ester

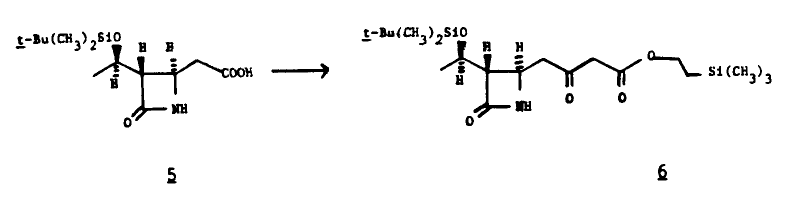

The title compound (754 mg) was prepared in 96% yield as a colorless oil by the method described in Example 5 using carboxylic acid 5 (prepared as described in U.S. Patent No. 4,499,278) instead of carboxylic acid 1, magnesium 2-(trimethylsilyl)ethyl malonate (prepared as described in Example 2) instead of magnesium 4-nitrobenzyl malonate, and tetrahydrofuran at room temperature instead of dimethylformamide at 50°. The reaction mixture was diluted with dichloromethane (40 ml), washed sequentially with dilute hydrochloric acid (ca. 25 ml) and water, dried over magnesium sulfate, filtered, and concentrated. Purification by flash chromatography used 1:1 ethyl acetate-hexane as eluent. Structure assignment was supported by the nmr and infrared spectra and by chemical ionization mass spectrometry:
- ¹H nmr (CDCl₃; 250 MHz):: δ (ppm) 0.05 (s, 9H); 0.06 (s, 3H); 0.07 (s, 3H); 0.87 (s, 9H); 1.01 (m, 2H); 1.20 (d, J=6.24 Hz, 3H); 2.77 (dd, J=2.6, 4.9 Hz, 1H); 2.78 (dd, J=9.7, 18.2 Hz, 1H); 3.05 (dd, J=3.2, 18.2 Hz, 1H); 3.44 (s, 2H); 3.96 (m, 1H); 4.18 (m, 1H); 4.23 (m, 2H); 6.10 (bs, 1H; NH).
- IR (thin film):: 3300, 2950, 1750, 1735, 1720, 1255, 835 cm⁻¹.
- MS (C.I.):: 402 (MH⁺), 386 (M-CH₃), 344 (M-C(CH₃)₃).

### Example 9

### [2α,3β(S*)]-3-(1-Hydroxyethyl)-β,4-dioxo-2-azetidinebutanoic acid 2-(trimethylsilyl)ethyl ester

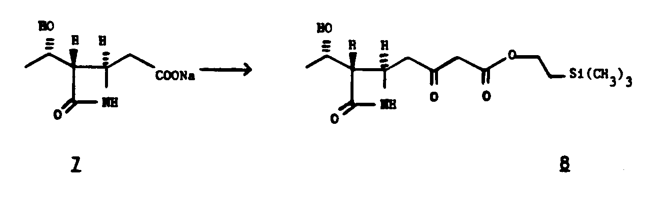

The title compound (460 mg) was prepared in 80% yield as a colorless oil by the method of Example 8 using the sodium salt 7 instead of the silyl protected compound 1 (see Example 5). The sodium salt 7 was prepared as described in U.S. Patent No. 4,350,631. Flash chromatography used ethyl acetate as eluent. Structure assignment was supported by the nmr and infrared spectra:
- ¹H nmr (CDCl₃; 250 MHz):: δ (ppm) 0.05 (s, 9H); 1.04 (m, 2H); 1.30 (d, J=6.45 Hz, 3H); 2.83 (dd, J=2.2, 7.0 Hz, 1H); 2.93 (dd, J=7.8, 18.3 Hz, 1H); 3:05 (dd, J=5.5, 18.3 Hz, 1H); 3.46 (s, 2H); 3.94 (m, 1H); 4.15 (m, 1H); 4.20 (m, 2H); 6.37 (bs, 1H; NH).
- IR (thin film):: 3350, 2960, 1745, 1725, 1710, 1260, 860, 840 cm⁻¹.

### Example 10

### [2α,3β(R*)]-3-[1-[[(1,1-Dimethylethyl)dimethylsilyl]oxy]ethyl]-β,4-dioxo-2-azetidinebutanoic acid 2,2,2-trichloroethyl ester

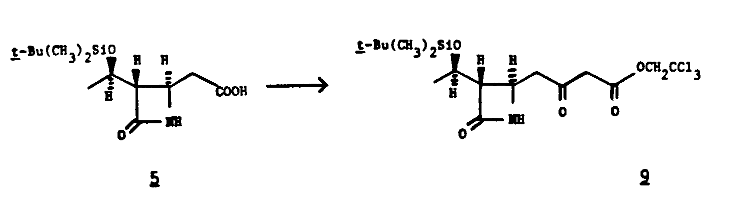

The title compound (820 mg) was prepared in 88% yield as a colorless oil by the method of Example 8 (see also Example 5) using magnesium 2,2,2-trichloroethyl malonate (prepared as described in Example 3) instead of magnesium 2-(trimethylsilyl)ethyl malonate. Structure assignment was supported by the nmr and infrared spectra and by chemical ionization mass spectrometry:
- ¹H nmr (CDCl₃; 250 MHz):: δ (ppm) 0.06 (s, 3H); 0.07 (s, 3H); 0.87 (s, 9H); 1.20 (d, J=6.24 Hz, 3H); 2.79 (dd, J=2.4, 4.9 Hz, 1H); 2.80 (dd, J=9.8, 18.2 Hz, 1H); 3.10 (dd, J=3.3, 18.2 Hz, 1H); 3.63 (s, 2H); 3.98 (m, 1H); 4.18 (m, 1H); 4.80 (s, 2H); 6.09 (bs, 1H; NH).
- IR (thin film):: 3250, 2950, 1755, 1745, 1720, 1625, 1255, 1140, 840 cm⁻¹.
- MS (C.I.):: 462 (MH⁺ + 2), 460 (MH⁺), 446, 444, 426, 424, 404, 402.

### Example 11

### [2α,3β(R*)]-3-[1-[[(1,1-Dimethylethyl)dimethylsilyl]oxy]ethyl]-β,4-dioxo-2-azetidinebutanoic acid 3-phenyl-2-propenyl ester

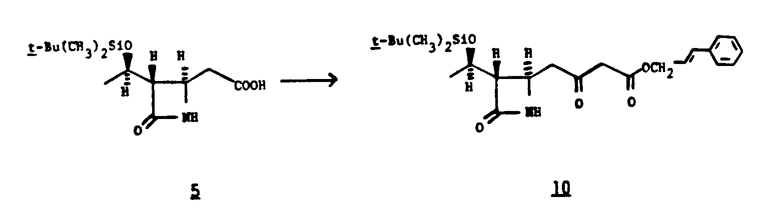

The title compound (930 mg) was prepared in 83% yield by the method of Example 8 (see also Example 5) using magnesium cinnamyl malonate (prepared as described in Example 4) instead of magnesium 2-(trimethylsilyl)ethyl malonate. Structure assignment was supported by the nmr and infrared spectra and by chemical ionization mass spectrometry:
- ¹H nmr (CDCl₃; 250 MHz):: δ (ppm) 0.07 (pair s, 6H); 0.87 (s, 9H); 1.20 (d, J=6.25 Hz, 3H); 2.76 (dd, J=3.0, 5.3 Hz, 1H); 2.80 (dd, J=9.9, 18.1 Hz, 1H); 3.06 (dd, J=3.3, 18.1 Hz, 1H); 3.52 (s, 2H); 3.99 (m, 1H); 4.17 (m, 1H); 4.80 (dd, J=1.3, 6.6 Hz, 2H); 6.11 (bs, 1H; NH); 6.28 (m, 1H); 6.68 (d, J=15.8 Hz, 1H); 7.41 (m, 5H).
- IR (thin film):: 2925, 2930, 1750, 1735, 1715, 1250, 1140, 1090, 965, 830 cm⁻¹.
- MS (C.I.):: 446 (MH⁺), 430 (M-CH₃), 388 (M-C(CH₃)₃).

### Example 12

### [2α,3β(R*)]-3-[1-[[1,1-Dimethylethyl)dimethylsilyl]oxy]ethyl]-β,4-dioxo-2-azetidinebutanoic acid (4-nitrophenyl)methyl ester

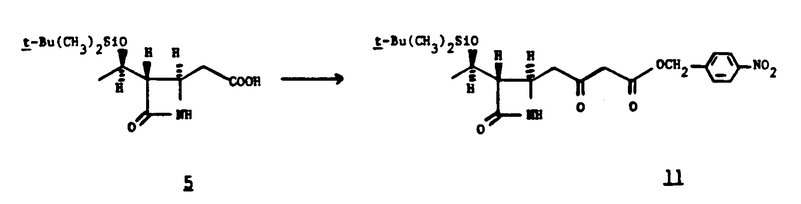

The title compound was prepared in 97% yield by the method of Example 5 using the carboxylic acid 5 instead of the carboxylic acid 1.

### Example 13

### [2α,3β(S*)]-3-[1-Hydroxyethyl)-β,4-dioxo-2-azetidinebutanoic acid (4-nitrophenyl)methyl ester

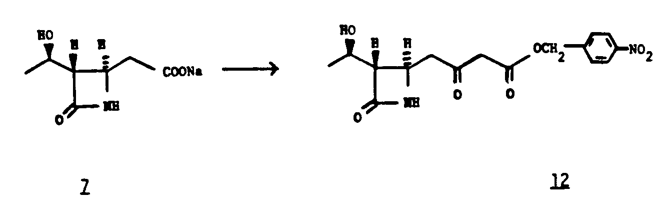

The title compound was prepared in 88% yield by the method of Example 5 using the sodium salt 7 instead of the carboxylic acid 1.

### Example 14

### [2α,3β(R*)]-3-[1-Hydroxyethyl)-β,4-dioxo-2-azetidinebutanoic acid (4-nitrophenyl)methyl ester

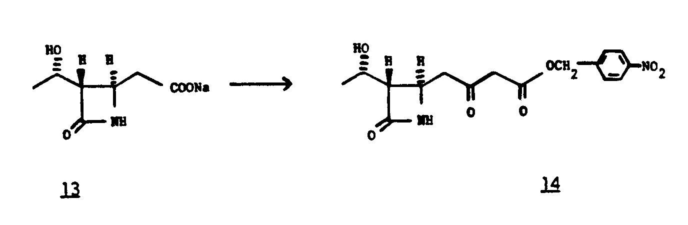

The title compound was prepared in 94% yield by the method of Example 5 using the sodium salt 13 instead of the carboxylic acid 1. The sodium salt 13 was prepared from the free carboxylic acid, which was prepared as described in U.S. Patent No. 4,499,278.

### Example 15

### [2α,3β(R*)]-3-[1-[[(1,1-Dimethylethyl)dimethylsilyl]oxy]ethyl]-β,4-dioxo-2-azetidinebutanoic acid 2-propenyl ester

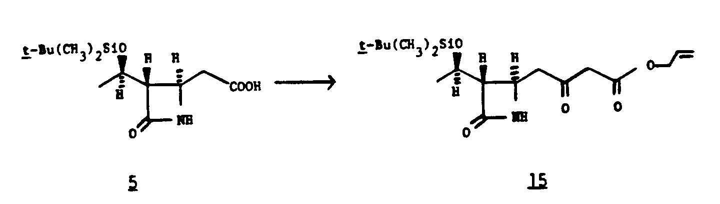

The title compound was prepared by the methods described in Examples 6 and 7 using the carboxylic acid 5 instead of the carboxylic acid 1.

## Claims

1. A process for preparing a compound of the formula 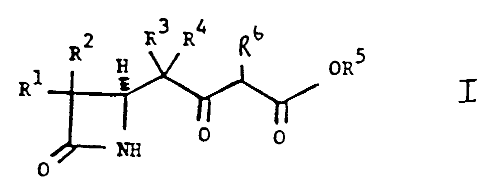 wherein R¹ and R² are independently:
a) hydrogen;
b) C₁-C₄ alkyl;
c) C₁-C₄ alkyl substituted at the 1-position with a substituent selected from the group consisting of:
i) hydroxy;
ii) tris(C₁-C₄ alkyl)silyloxy; or
iii) allyloxycarbonyloxy; or
d) fluorinated C₁-C₄ alkyl; with the proviso that at least one of R¹ and R² is hydrogen;
R³ and R⁴ are independently:
a) hydrogen; or
b) C₁-C₄ alkyl;
R⁵ is:
a) benzyl or benzyl substituted in the benzene ring with one to three substituents selected from the group consisting of:
i) NO₂;
ii) halogen; or
iii) C₁-C₄ alkyl;
b) C₁-C₄ alkyl or C₁-C₄ alkyl
substituted with one or more substituents selected from the group consisting of:
i) halogen; or
ii) tris(C₁-C₄ alkyl)silyl; or
c) C₃-C₆ alkenyl or C₃-C₆ alkenyl
substituted with a substituent selected from the group consisting of:
i) phenyl;
ii) naphthyl; or
iii) biphenylyl; and
R⁶ is:
a) hydrogen; or
b) COOR⁷ such that R⁵ and R⁷ taken together are -C(CH₃)₂-;
comprising:
a) reacting at least about one part to about three parts by moles of a malonate derivative of the formula 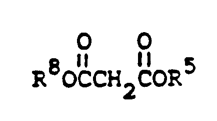 wherein R⁵ is defined as above and R⁸ is hydrogen or wherein R⁵ and R⁸ taken together are -C(CH₃)₂-,
with about one part by moles of a magnesium dihalide and about two parts by moles of an amine base in an organic solvent; and
b) adding about one part to about two parts by moles of an activated acyl compound of the formula 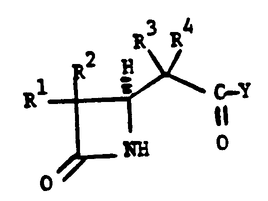 wherein R¹, R², R³ and R⁴ are defined as above and Y is halogen, 1-imidazolyl, or C₁-C₅ acyloxy.

2. A process according to Claim 1 for preparing a compound of the formula I
comprising:
a) reacting at least about one part to about three parts by moles of a malonate derivative of the formula 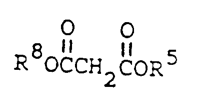 wherein R⁵ is defined in claim 1 and R⁸ is hydrogen or wherein R⁵ and R⁸ taken together are -C(CH₃)₂-,
with about one part by moles of magnesium chloride and about two parts by moles of triethylamine in tetrahydrofuran, dimethylformamide, or acetonitrile; and
b) adding about one part to about two parts by moles of an activated acyl compound of the formula 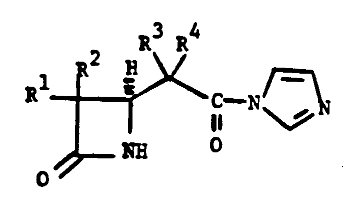 wherein R¹, R², R³, and R⁴ are defined in claim 1.

3. A process according to Claim 2 wherein R⁶ is hydrogen; R⁸ is hydrogen; one of R¹ or R² is hydrogen and the other of R¹ or R² is 1-hydroxyethyl; and R⁵ is 4-nitrobenzyl.

4. A process for preparing a compound of the formula 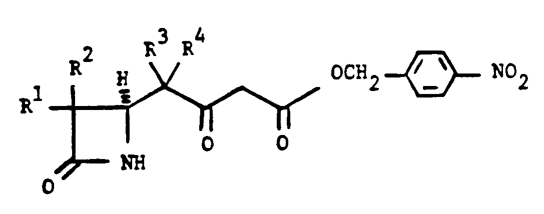 wherein one of R¹ or R² is hydrogen and the other of R¹ or R² is 1-hydroxyethyl; and R³ and R⁴ are independently hydrogen or C₁-C₄ alkyl;
comprising:
a) reacting about two parts by moles of a malonate derivative of the formula 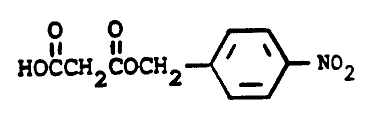 with about one part by moles of magnesium chloride and about two parts by moles of triethylamine in tetrahydrofuran or dimethylformamide; and
b) adding about one part by moles of an activated acyl compound of the formula 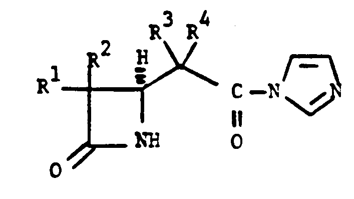 wherein R¹, R², R³, and R⁴ are defined as above.

5. A process for preparing a compound of the formula 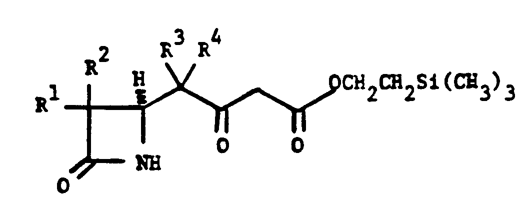 wherein one of R¹ or R² is hydrogen and the other of R¹ or R² is 1-hydroxyethyl; and R³ and R⁴ are independently hydrogen or C₁-C₄ alkyl;
comprising:
a) reacting about two parts by moles of a malonate derivative of the formula 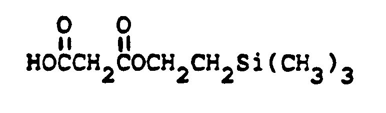 with about one part by moles of magnesium chloride and about two parts by moles of triethylamine in tetrahydrofuran or dimethylformamide; and
b) adding about one part by moles of an activated acyl compound of the formula 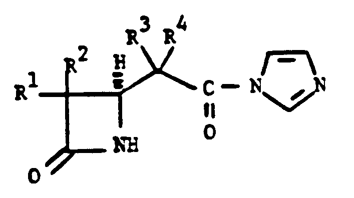 wherein R¹, R², R³, and R⁴ are defined as above.

6. A process for preparing a compound of the formula 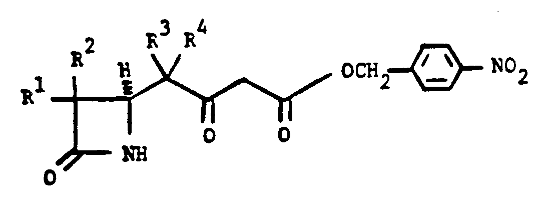 wherein one of R¹ or R² is hydrogen and the other of R¹ or R² is 1-(t-butyldimethylsilyloxy)ethyl; and R³ and R⁴ are independently hydrogen or C₁-C₄ alkyl;
comprising:
a) reacting about two parts by moles of a malonate derivative of the formula 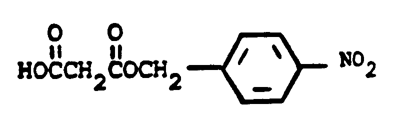 with about one part by moles of magnesium chloride and about two parts by moles of triethylamine in tetrahydrofuran or dimethylformamide; and
b) adding about one part by moles of an activated acyl compound of the formula 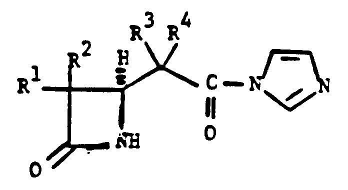 wherein R¹, R², R³, and R⁴ are defined as above.

7. A process for preparing a compound of the formula 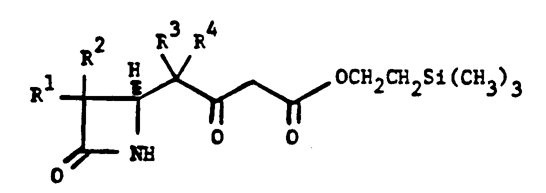 wherein one of R¹ or R² is hydrogen and the other of R¹ or R² is 1-(t-butyldimethylsilyloxy)ethyl; and
R³ and R⁴ are independently hydrogen or C₁-C₄ alkyl;
comprising:
a) reacting about two parts by moles of a malonate derivative of the formula 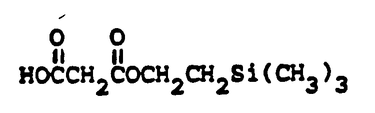 with about one part by moles of magnesium chloride and about two parts by moles of triethylamine in tetrahydrofuran or dimethylformamide; and
b) adding about one part by moles of an activated acyl compound of the formula 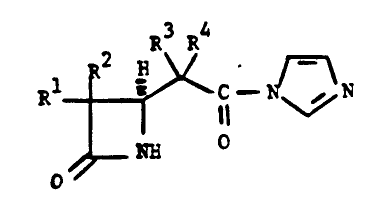 wherein R¹, R², R³, and R⁴ are defined as above.

8. A process for preparing a compound of the formula 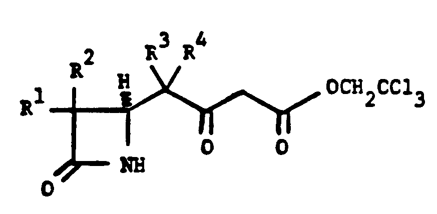 wherein one of R¹ or R² is hydrogen and the other of R¹ or R² is 1-(t-butyldimethylsilyloxy)ethyl; and
R³ and R⁴ are independently hydrogen or C₁-C₄ alkyl;
comprising:
a) reacting about two parts by moles of a malonate derivative of the formula 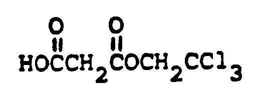 with about one part by moles of magnesium chloride and about two parts by moles of triethylamine in tetrahydrofuran or dimethylformamide; and
b) adding about one part by moles of an activated acyl compound of the formula 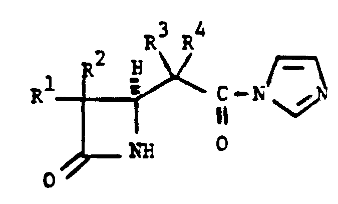 wherein R¹, R², R³, and R⁴ are defined as above.

9. A process preparing a compound of the formula 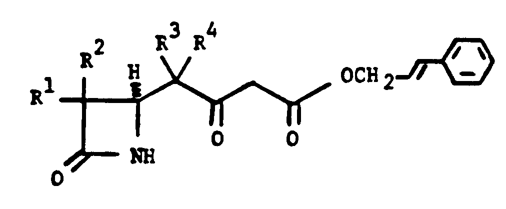 wherein one of R¹ or R² is hydrogen and the other of R¹ or R² is 1-(t-butyldimethylsilyloxy)ethyl; and
R³ and R⁴ are independently hydrogen or C₁-C₄ alkyl;
comprising:
a) reacting about two parts by moles of a malonate derivative of the formula 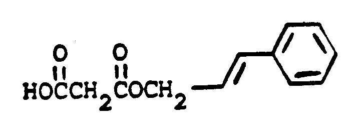 with about one part by moles of magnesium chloride and about two parts by moles of triethylamine in tetrahydrofuran or dimethylformamide; and
b) adding about one part by moles of an activated acyl compound of the formula 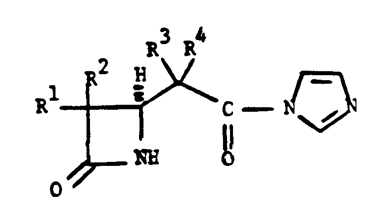 wherein R¹, R², R³, and R⁴ are defined as above.

10. A process for preparing a compound of the formula 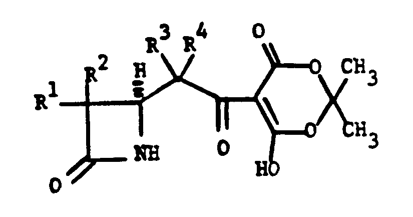 wherein R¹ and R² are independently:
a) hydrogen;
b) C₁-C₄ alkyl;
c) C₁-C₄ alkyl substituted at the 1-position with a substituent selected from the group consisting of:
i) hydroxy;
ii) tris(C₁-C₄ alkyl)silyloxy; or
iii) allyloxycarbonyloxy; or
d) fluorinated C₁C₄ alkyl; with the proviso that at least one of R¹ and R² is hydrogen; and
R³ and R⁴ are independently:
a) hydrogen; or
b) C₁-C₄ alkyl;
comprising:
a) reacting at least about one part by moles of a malonate derivative of the formula 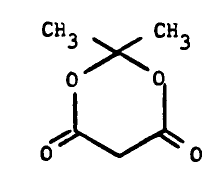 with about one part by moles of magnesium chloride and about two parts by moles of triethylamine in tetrahydrofuran, dimethylformamide, or acetonitrile; and
b) adding about one part by moles of an activated acyl compound of the formula 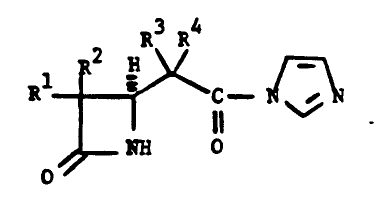 wherein R¹, R², R³, and R⁴ are defined as above.

11. A process for preparing a compound of the formula 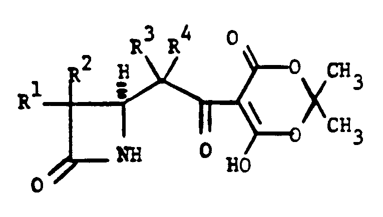 wherein one of R¹ or R² is hydrogen and the other of R¹ or R² is 1-(t-butyldimethylsilyloxy)ethyl; and
R³ and R⁴ are independently hydrogen or C₁-C₄ alkyl;
comprising:
a) reacting at least about one part by moles of a malonate derivative of the formula 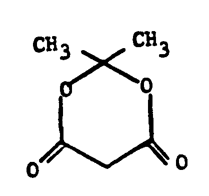 with about one part by moles of magnesium chloride and about two parts by moles of triethylamine in acetonitrile; and
b) adding about one part by moles of an activated acyl compound of the formula 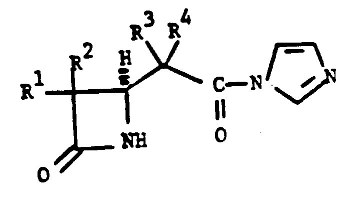 wherein R¹, R², R³, and R⁴ are defined as above.

## Patentansprüche

1. Verfahren zur Herstellung einer Verbindung der Formel 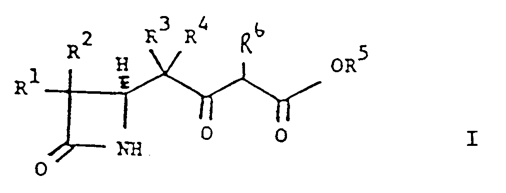 in der R¹ und R² unabhängig voneinander
a) Wasserstoff,
b) C₁-C₄ Alkyl,
c) in 1-Stellung durch einen Substituenten, ausgewählt aus der Gruppe von
i) Hydroxyl,
ii) tris-(C₁-C₄ Alkyl)-silyloxy,
iii) Allyloxycarbonyloxy,
substituiertes C₁-C₄ Alkyl, oder
d) fluoriertes C₁-C₄ Alkyl, wobei jedoch mindestens einer der Reste R¹ und R² Wasserstoff sein muß,
R³ und R⁴ unabhängig voneinander
a) Wasserstoff oder
b) C₁-C₄ Alkyl,
R⁵
a) Benzyl oder am Benzolring mit einem der drei Substituenten, ausgewählt aus der Gruppe von
i) NO₂,
ii) Halogen oder
iii) C₁-C₄ Alkyl,
substituiertes Benzyl,
b) C₁-C₄ Alkyl oder mit einem oder mehreren Substituenten ausgewählt aus der Gruppe von
i) Halogen oder
ii) tris-(C₁-C₄ Alkyl)-silyl
substituiertes C₁-C₄ Alkyl, oder
c) C₃-C₆ Alkenyl oder mit einem Substituenten ausgewählt aus der Gruppe von
i) Phenyl,
ii) Naphthyl oder
iii) Biphenylyl
substituiertes C₃-C₆ Alkenyl
und
R⁶
a) Wasserstoff oder
b) COOR⁷, wobei R⁵ und R⁷ zusammen die Gruppe -C(CH₃)₂- bilden,
bedeuten, umfassend
a) Umsetzung von zumindest etwa einem Molteil bis etwa drei Molteilen eines Malonsäurederivats der Formel 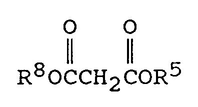 in der R⁵ die obigen Bedeutungen besitzt und R⁸ Wasserstoff ist oder worin R⁵ und R⁸ zusammen die Gruppe -C(CH₃)₂- bilden,
mit etwa einem Molteil eines Magnesiumdihalogenids und mit etwa zwei Molteilen einer Aminbase in einem organischen Lösungsmittel, und
b) Zugabe von etwa ein bis etwa zwei Molteilen einer aktivierten Acylverbindung der Formel 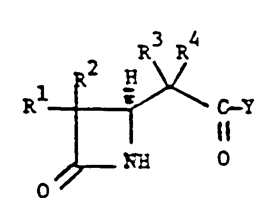 in der R¹, R², R³ und R⁴ die obigen Bedeutungen besitzen und Y Halogen, 1-Imidazolyl oder C₁-C₅ Acyloxy bedeuten.

2. Verfahren gemäß Anspruch 1 zur Herstellung einer Verbindung der Formel I,
umfassend
a) Umsetzung von zumindest etwa einem Molteil bis etwa drei Molteilen eines Malonsäurederivats der Formel 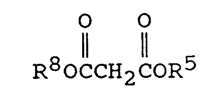 in der R⁵ die im Anspruch 1 genannten Bedeutungen besitzt und R⁸ Wasserstoff ist oder worin R⁵ und R⁸ zusammen die Gruppe -C(CH₃)₂- bilden,
mit etwa einem Molteil Magnesiumchlorid und mit etwa zwei Molteilen von Triethylamin in Tetrahydrofuran, Dimethylformamid oder Acetonitril, und
b) Zugabe von etwa einem bis etwa zwei Molteilen einer aktivierten Acylverbindung der Formel 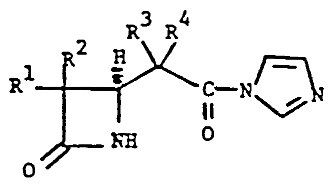 in der R¹, R², R³ und R⁴ die im Anspruch 1 genannten Bedeutungen besitzen.

3. Verfahren gemäß Anspruch 2, worin R⁶ Wasserstoff, R⁸ Wasserstoff, einer der Reste R¹ oder R² Wasserstoff und der andere der Reste R¹ oder R² 1-Hydroxyethyl und R⁵ 4-Nitrobenzyl bedeuten.

4. Verfahren zur Herstellung einer Verbindung der Formel 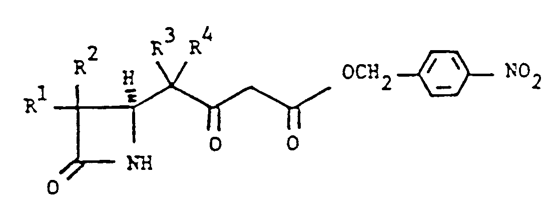 in der einer der Reste R¹ oder R² Wasserstoff und der andere der Reste R¹ oder R² Hydroxyethyl, und R³ und R⁴ unabhängig voneinander Wasserstoff oder C₁-C₄ Alkyl bedeuten,
umfassend
a) Umsetzung von etwa zwei Molteilen eines Malonsäurederivats der Formel 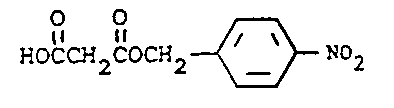 mit etwa einem Molteil Magnesiumchlorid und etwa zwei Molteilen Triethylamin in Tetrahydrofuran oder Dimethylformamid, und
b) Zugabe von etwa einem Molteil einer aktivierten Acylverbindung der Formel 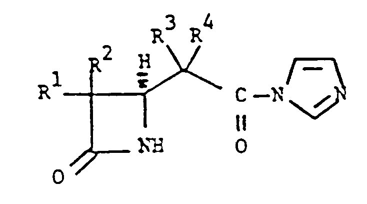 in der R¹, R², R³ und R⁴ die obigen Bedeutungen aufweisen.

5. Verfahren zur Herstellung einer Verbindung der Formel 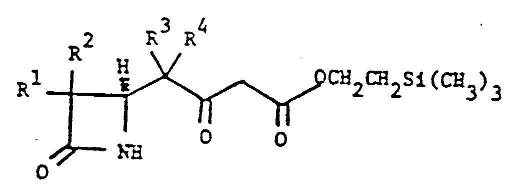 in der einer der Reste R¹ oder R² Wasserstoff und der andere der Reste R¹ oder R² Hydroxyethyl, und R³ und R⁴ unabhängig voneinander Wasserstoff oder C₁-C₄ Alkyl bedeuten,
umfassend
a) Umsetzung von etwa zwei Molteilen eines Malonsäurederivats der Formel 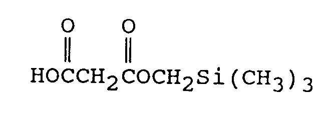 mit etwa einem Molteil Magnesiumchlorid und etwa zwei Molteilen Triethylamin in Tetrahydrofuran oder Dimethylformamid, und
b) Zugabe von etwa einem Molteil einer aktivierten Acylverbindung der Formel 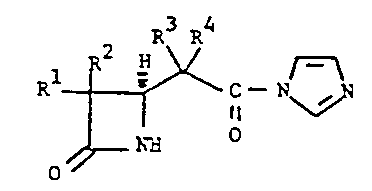 in der R¹, R², R³ und R⁴ die obigen Bedeutungen aufweisen.

6. Verfahren zur Herstellung einer Verbindung der Formel 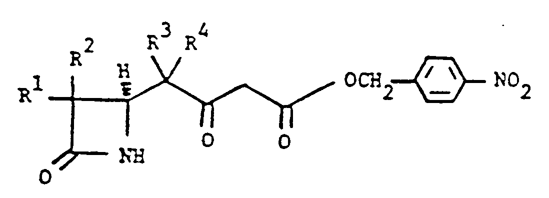 in der einer der Reste R¹ oder R² Wasserstoff und der andere der Reste R¹ oder R² 1-(tert.Butyldimethylsilyloxy)-ethyl, und R³ und R⁴ unabhängig voneinander Wasserstoff oder C₁-C₄ Alkyl bedeuten,
umfassend
a) Umsetzung von etwa zwei Molteilen eines Malonsäurederivats der Formel 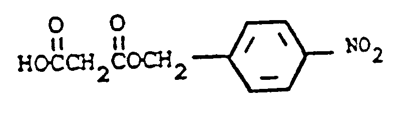 mit etwa einem Molteil Magnesiumchlorid und etwa zwei Molteilen Triethylamin in Tetrahydrofuran oder Dimethylformamid, und
b) Zugabe von etwa einem Molteil einer aktivierten Acylverbindung der Formel 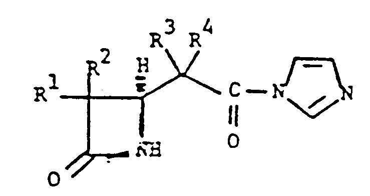 in der R¹, R², R³ und R⁴ die obigen Bedeutungen aufweisen.

7. Verfahren zur Herstellung einer Verbindung der Formel 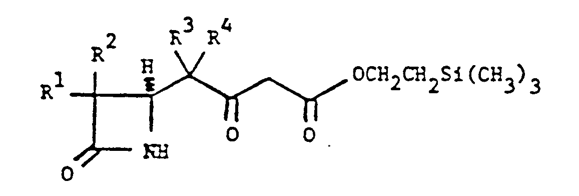 in der einer der Reste R¹ oder R² Wasserstoff und der andere der Reste R¹ oder R² 1-(tert.Butyldimethylsilyloxy)-ethyl, und R³ und R⁴ unabhängig voneinander Wasserstoff oder C₁-C₄ Alkyl bedeuten,
umfassend
a) Umsetzung von etwa zwei Molteilen eines Malonsäurederivats der Formel 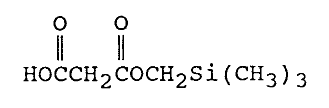 mit etwa einem Molteil Magnesiumchlorid und etwa zwei Molteilen Triethylamin in Tetrahydrofuran oder Dimethylformamid, und
b) Zugabe von etwa einem Molteil einer aktivierten Acylverbindung der Formel 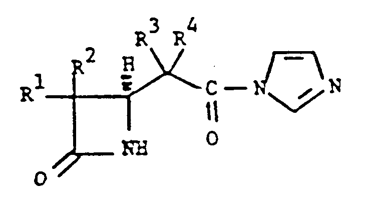 in der R¹, R², R³ und R⁴ die obigen Bedeutungen aufweisen.

8. Verfahren zur Herstellung einer Verbindung der Formel 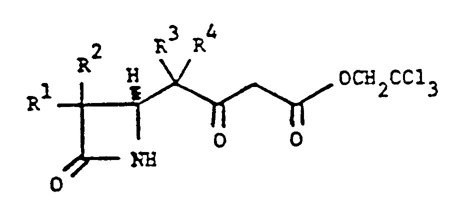 in der einer der Reste R¹ oder R² Wasserstoff und der andere der Reste R¹ oder R² 1-(tert.Butyldimethylsilyloxy)-ethyl, und R³ und R⁴ unabhängig voneinander Wasserstoff oder C₁-C₄ Alkyl bedeuten,
umfassend
a) Umsetzung von etwa zwei Molteilen eines Malonsäurederivats der Formel 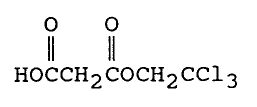 mit etwa einem Molteil Magnesiumchlorid und etwa zwei Molteilen Triethylamin in Tetrahydrofuran oder Dimethylformamid, und
b) Zugabe von etwa einem Molteil einer aktivierten Acylverbindung der Formel 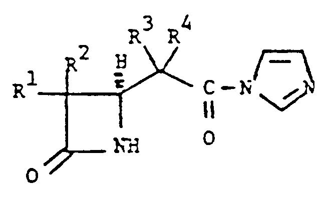 in der R¹, R², R³ und R⁴ die obigen Bedeutungen aufweisen.

9. Verfahren zur Herstellung einer Verbindung der Formel 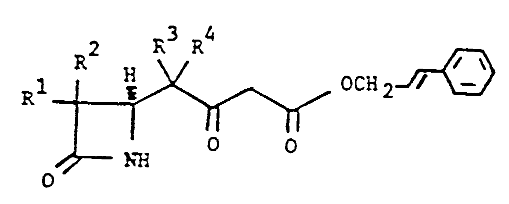 in der einer der Reste R¹ oder R² Wasserstoff und der andere der Reste R¹ oder R² 1-(tert.Butyldimethylsilyloxy)-ethyl, und R³ und R⁴ unabhängig voneinander Wasserstoff oder C₁-C₄ Alkyl bedeuten,
umfassend
a) Umsetzung von etwa zwei Molteilen eines Malonsäurederivats der Formel 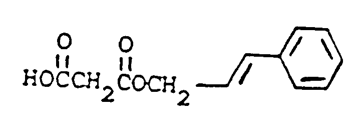 mit etwa einem Molteil Magnesiumchlorid und etwa zwei Molteilen Triethylam in Tetrahydrofuran oder Dimethylformamid, und
b) Zugabe von etwa einem Molteil einer aktivierten Acylverbindung der Formel 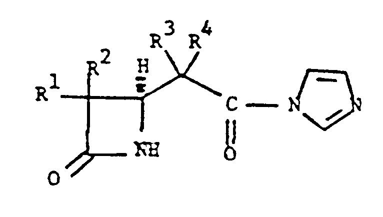 in der R¹, R², R³ und R⁴ die obigen Bedeutungen aufweisen.

10. Verfahren zur Herstellung einer Verbindung der Formel 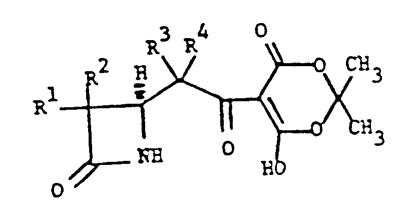 in der R¹ und R² unabhängig voneinander
a) Wasserstoff,
b) C₁-C₄ Alkyl
c) in 1-Stellung mit einem Substituenten, ausgewählt aus der Gruppe von
i) Hydroxyl,
ii) tris-(C₁-C₄ Alkyl)silyloxy oder
iii) Allyloxycarbonyloxy, oder
substituiertes C₁-C₄ Alkyl
d) fluoriertes C₁-C₄ Alkyl, wobei jedoch mindestens einer der Reste R¹ oder R² Wasserstoff sein muß,
und R³ und R⁴ unabhängig voneinander
a) Wasserstoff oder
b) C₁-C₄ Alkyl bedeuten,
umfassend
a) Umsetzung von etwa zwei Molteilen eines Malonsäurederivats der Formel 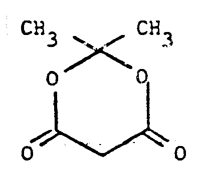 mit etwa einem Molteil Magnesiumchlorid und etwa zwei Molteile Triethylamin in Tetrahydrofuran oder Dimethylformamid, und
b) Zugabe von etwa einem Molteil einer aktivierten Acylverbindung der Formel 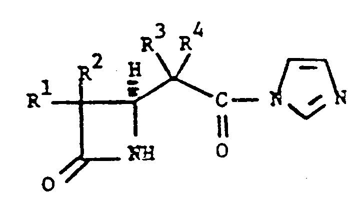 in der R¹, R², R³ und R⁴ die obigen Bedeutungen aufweisen.

11. Verfahren zur Herstellung einer Verbindung der Formel 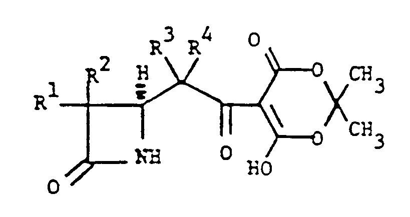 in der einer der Reste R¹ oder R² Wasserstoff und der andere der Reste R¹ oder R² 1-(tert.Butyldimethylsilyloxy)-ethyl, und R³ und R⁴ unabhängig voneinander Wasserstoff oder C₁-C₄ Alkyl bedeuten,
umfassend
a) Umsetzung von etwa zwei Molteilen eines Malonsäurederivats der Formel 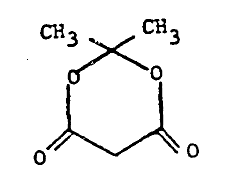 mit etwa einem Molteile Magnesiumchlorid und etwa zwei Molteilen Triethylamin in Tetrahydrofuran oder Dimethylformamid, und
b) Zugabe von etwa einem Molteil einer aktivierten Acylverbindung der Formel 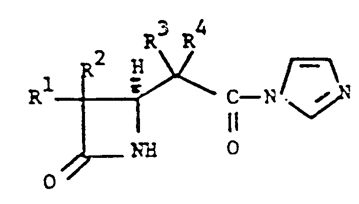 in der R¹, R², R³ und R⁴ die obigen Bedeutungen aufweisen.

## Revendications

1. Procédé de préparation d'un composé de formule 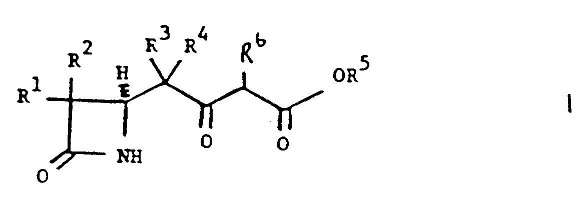 dans laquelle R¹ et R² représentent indépendamment :
a) un hydrogène ;
b) un alkyle en C₁-C₄ ;
c) un alkyle en C₁-C₄ substitué en position 1 avec un substituant choisi dans le groupe constitué par :
i) un hydroxy
ii) un tris(alkyle en C₁-C₄)silyloxy ; ou
iii) un allyloxycarbonyloxy ; ou
d) un alkyle en C₁-C₄ fluoré ; à condition qu'au moins l'un des R¹ et R² soit un hydrogène ;
R³ et R⁴ représentent indépendamment :
a) un hydrogène; ou
b) un alkyle en C₁-C₄ ;
R⁵ représente :
a) un benzyle ou un benzyle substitué sur le cycle benzènique avec de un à trois substituants choisis dans le groupe consistant en :
i) NO₂ ;
ii) un halogène ;
iii) un alkyle en C₁-C₄ ;
b) un alkyle en C₁-C₄ ou un alkyle en C₁-C₄ substitué avec un ou plusieurs substituants choisis dans le groupe constitué par :
i) un halogène ; ou
ii) un tris(alkyle en C₁-C₄)silyle ; ou
c) un alkènyle en C₃-C₆ ou un alkènyle en C₃-C₆ substitué avec un substituant choisi dans le groupe constitué par :
i) un phényle ;
ii) un naphtyle
iii) un biphénylyle ; et
R⁶ représente :
a) un hydrogène ; ou
b) COOR⁷ tel que R⁵ et R⁷ pris ensemble forment -C(CH₃)₂-.;
comprenant :
a) la réaction d'au moins environ une part jusqu'à environ trois parts, en moles, d'un dérivé de malonate de formule 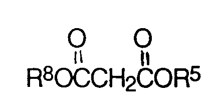 dans laquelle R⁵ est défini comme ci-dessus et R⁸ est un hydrogène ou dans laquelle R⁵ et R⁸ pris ensemble sont -C(CH₃)₂-,
avec environ une part, en moles, d'un dihalogénure de magnésium et environ deux parts, en moles, d'une amine basique dans un solvant organique ;
b) l'addition d'entre environ une part et environ deux parts, en moles, d'un composé acylé activé de formule 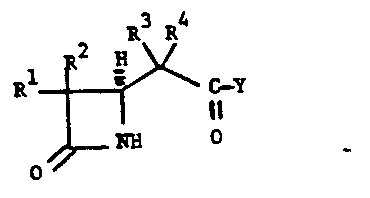 dans laquelle R¹, R², R³, et R⁴ sont comme définis ci-dessus et Y est un halogène, un 1-imidazolyle, ou un acyloxy en C₁-C₅.

2. Un procédé selon la revendication 1, de préparation d'un composé de formule I, comprenant :
a) de faire réagir entre au moins environ une part et environ trois parts, en moles, d'un dérivé de malonate de formule 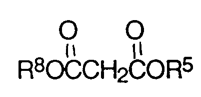 dans laquelle R⁵ est défini comme à la revendication 1 et R⁸ est un hydrogène ou dans laquelle R⁵ et R⁸ pris ensemble représentent -C(CH₃)₂-,
avec environ une part, en moles, de chlorure de magnésium et environ deux parts, en moles, de triéthylamine dans du tétrahydrofurane, de l'acétonitrile ou du diméthylformamide ; et
b) d'ajouter entre environ une part et environ deux parts, en moles, d'un composé acylé activé de formule 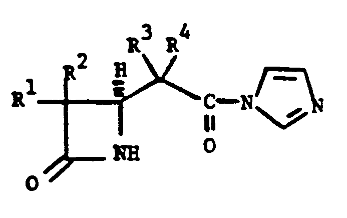 dans laquelle R¹, R², R³, et R⁴ sont comme définis à la revendication 1.

3. Procédé selon la revendication 2 dans lequel R⁶ est un hydrogène ; R⁸ est un hydrogène ; l'un des deux substituants R¹ et R² représente un hydrogène et l'autre substituant représente un groupe 1-hydroxyéthyle ; et R⁵ est un groupe 4-nitrobenzyle.

4. Procédé de préparation d'un composé de formule 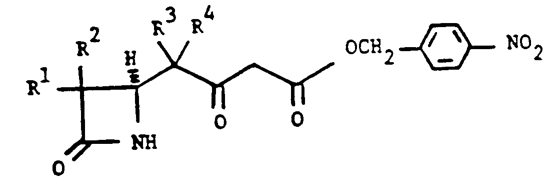 dans laquelle l'un des deux substituants R¹ et R² représente un hydrogène et l'autre substituant représente un groupe 1-hydroxyéthyle ; et R³ et R⁴ représentent indépendamment un hydrogène ou un alkyle en C₁-C₄ ;
comprenant :
a) de faire réagir environ deux parts, en moles, d'un dérivé de malonate de formule 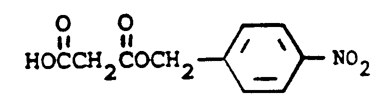 avec environ une part, en moles, de chlorure de magnésium et environ deux parts, en moles, de triéthylamine dans du tétrahydrofurane ou du diméthylformamide ; et
b) d'ajouter environ une part, en moles, d'un composé acylé activé de formule 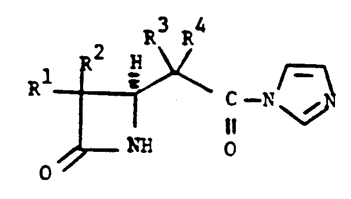 dans laquelle R¹, R², R³, et R⁴ sont comme définis ci-dessus.

5. Procédé de préparation d'un composé de formule 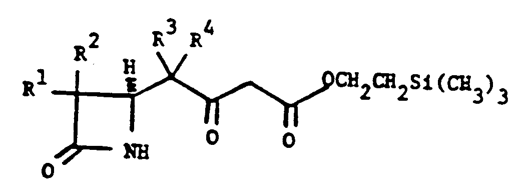 dans laquelle l'un des deux substituants R¹ et R² représente un hydrogène et l'autre substituant représente un groupe 1-hydroxyéthyle ; et R³ et R⁴ représentent indépendamment un hydrogène ou un alkyle en C₁-C₄ ;
comprenant :
a) de faire réagir environ deux parts, en moles, d'un dérivé de malonate de formule 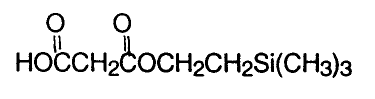 avec environ une part, en moles, de chlorure de magnésium et environ deux parts, en moles, de triéthylamine dans du tétrahydrofurane ou du diméthylformamide ; et
b) d'ajouter environ une part, en moles, d'un composé acylé activé de formule 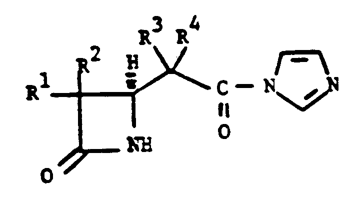 dans laquelle R¹, R², R³, et R⁴ sont comme définis ci-dessus.

6. Procédé de préparation d'un composé de formule 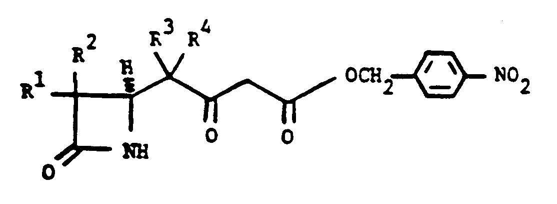 dans laquelle l'un des deux substituants R¹ et R² représente un hydrogène et l'autre substituant représente un groupe 1-(t-butyldiméthylsilyloxy)éthyle ; et R³ et R⁴ représentent indépendamment un hydrogène ou un alkyle en C₁-C₄ ;
comprenant :
a) de faire réagir environ deux parts, en moles, d'un dérivé de malonate de formule 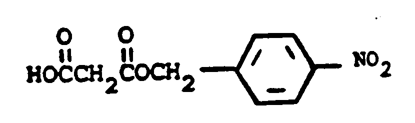 avec environ une part, en moles, de chlorure de magnésium et environ deux parts, en moles, de triéthylamine dans du tétrahydrofurane ou du diméthylformamide ; et
b) d'ajouter environ une part, en moles, d'un composé acylé activé de formule 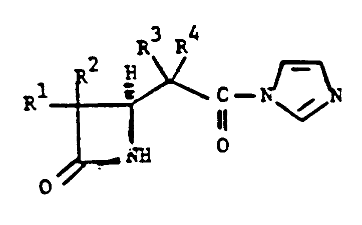 dans laquelle R¹, R², R³, et R⁴ sont comme définis ci-dessus.

7. Procédé de préparation d'un composé de formule 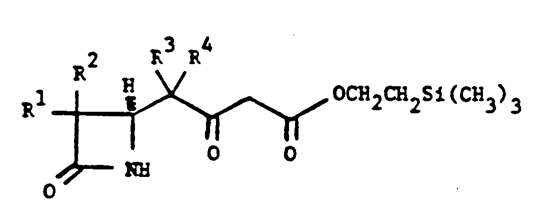 dans laquelle l'un des deux substituants R¹ et R² représente un hydrogène et l'autre substituant représente un groupe 1-(t-butyldiméthylsilyloxy)éthyle ; et
R³ et R⁴ représentent indépendamment un hydrogène ou un alkyle en C₁-C₄ ;
comprenant :
a) de faire réagir environ deux parts, en moles, d'un dérivé de malonate de formule 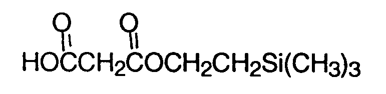 avec environ une part, en moles, de chlorure de magnésium et environ deux parts, en moles, de triéthylamine dans du tétrahydrofurane ou du diméthylformamide ; et
b) d'ajouter environ une part, en moles, d'un composé acylé activé de formule 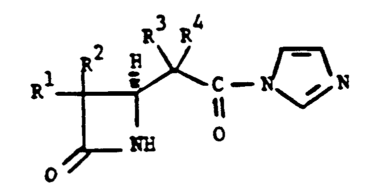 dans laquelle R¹, R², R³, et R⁴ sont comme définis ci-dessus.

8. Procédé de préparation d'un composé de formule 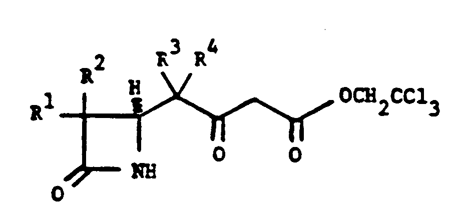 dans laquelle l'un des deux substituants R¹ et R² représente un hydrogène et l'autre substituant représente un groupe 1-(t-butyldiméthylsilyloxy)éthyle ; et
R³ et R⁴ représentent indépendamment un hydrogène ou un alkyle en C₁-C₄ ;
comprenant :
a) de faire réagir environ deux parts, en moles, d'un dérivé de malonate de formule 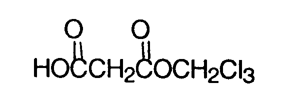 avec environ une part, en moles, de chlorure de magnésium et environ deux parts, en moles, de triéthylamine dans du tétrahydrofurane ou du diméthylformamide ; et
b) d'ajouter environ une part, en moles, d'un composé acylé activé de formule 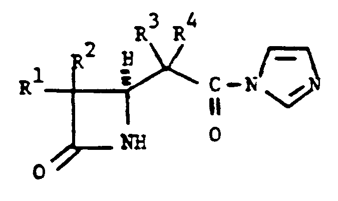 dans laquelle R¹, R², R³, et R⁴ sont comme définis ci-dessus.

9. Procédé de préparation d'un composé de formule 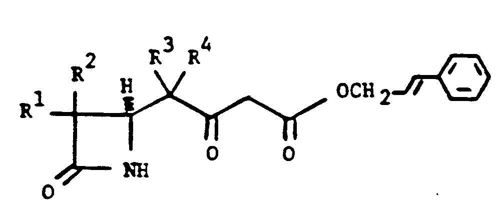 dans laquelle l'un des deux substituants R¹ et R² représente un hydrogène et l'autre substituant représente un groupe 1-(t-butyldiméthylsilyloxy)éthyle ; et
R³ et R⁴ représentent indépendamment un hydrogène ou un alkyle en C₁-C₄ ;
comprenant :
a) de faire réagir environ deux parts, en moles, d'un dérivé de malonate de formule 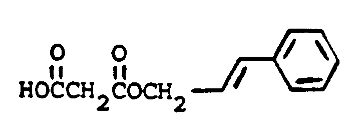 avec environ une part, en moles, de chlorure de magnésium et environ deux parts, en moles, de triéthylamine dans du tétrahydrofurane ou du diméthylformamide ; et
b) d'ajouter environ une part, en moles, d'un composé acylé activé de formule 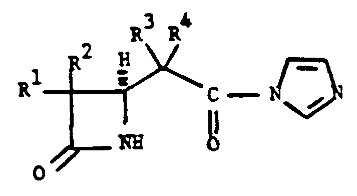 dans laquelle R¹, R², R³, et R⁴ sont comme définis ci-dessus.

10. Procédé de préparation d'un composé de formule 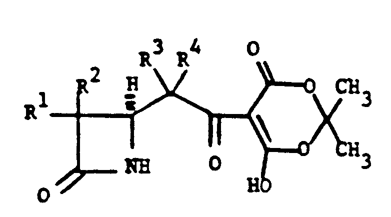 dans laquelle R¹ et R² représentent indépendamment :
a) un hydrogène ;
b) un alkyle en C₁-C₄ ;
c) un alkyle en C₁-C₄ substitué en position 1 avec un substituant choisi dans le groupe constitué par :
i) un hydroxy
ii) un tris(alkyle en C₁-C₄)silyloxy ; ou
iii) un allyloxycarbonyloxy ; ou
d) un alkyle en C₁-C₄ fluoré ; à condition qu'au moins l'un des R¹ et R² soit un hydrogène ;
R³ et R⁴ représentent indépendamment :
a) un hydrogène ; ou
b) un alkyle en C₁-C₄ ;
comprenant :
a) de faire réagir au moins environ une part, en moles, d'un dérivé de malonate de formule 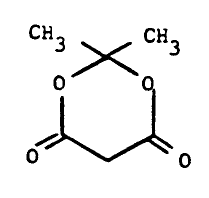 avec environ une part, en moles, de chlorure de magnésium et environ deux parts, en moles, de triéthylamine dans du tétrahydrofurane, du diméthylformamide ou de l'acétonitrile ; et
b) d'ajouter environ une part, en moles, d'un composé acylé activé de formule 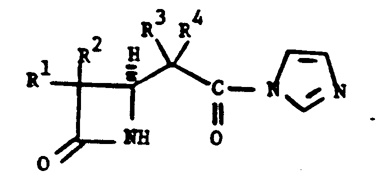 dans laquelle R¹, R², R³, et R⁴ sont comme définis ci-dessus.

11. Procédé de préparation d'un composé de formule 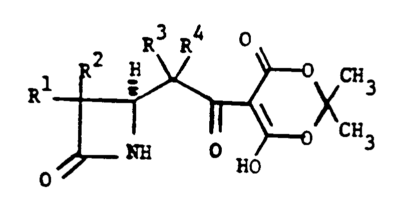 dans laquelle l'un des deux substituants R¹ et R² représente un hydrogène et l'autre substituant représente un groupe 1-(t-butyldiméthylsilyloxy)éthyle ; et
R³ et R⁴ représentent indépendamment un hydrogène ou un alkyle en C₁-C₄ ;
comprenant :
a) de faire réagir au moins environ une part, en moles, d'un dérivé de malonate de formule 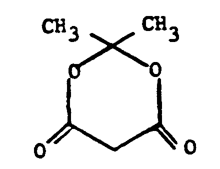 avec environ une part, en moles, de chlorure de magnésium et environ deux parts, en moles, de triéthylamine dans de l'acétonitrile ; et
b) d'ajouter environ une part, en moles, d'un composé acylé activé de formule 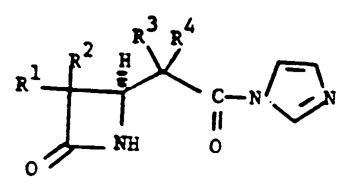 dans laquelle R¹, R², R³, et R⁴ sont comme définis ci-dessus.
